Europäisches Patentamt

European Patent Office

Office européen des brevets

(19)

(11) Veröffentlichungsnummer : **0 137 005**
**B1**

(12)   EUROPÄISCHE PATENTSCHRIFT

(45) Veröffentlichungstag der Patentschrift :
06.07.88

(21) Anmeldenummer : 84900716.6

(22) Anmeldetag : 08.02.84

(86) Internationale Anmeldenummer :
PCT/DE 84/00034

(87) Internationale Veröffentlichungsnummer :
WO/8403049 (16.08.84 Gazette 84/20)

(51) Int. Cl.⁴ : **A 61 N   5/06**, F 21 V 29/00

(54) LUFTGEKÜHLTES GERÄT ZUR BESTRAHLUNG MIT POLARISIERTEM LICHT.

(30) Priorität : 08.02.83 DE 3304230

(43) Veröffentlichungstag der Anmeldung :
17.04.85 Patentblatt 85/16

(45) Bekanntmachung des Hinweises auf die Patenterteilung : 06.07.88 Patentblatt 88/27

(84) Benannte Vertragsstaaten :
AT BE CH DE FR GB LI LU NL SE

(56) Entgegenhaltungen :
EP-A- 0 026 239
DE-A-   900 118
DE-A- 3 123 378
FR-A- 2 511 877
GB-A- 1 197 928
US-A- 3 185 028
US-A- 3 691 365
US-A- 3 936 672
US-A- 4 321 659

(73) Patentinhaber : AMS AUTOMATISCHE MESS- UND
STEUERUNGSTECHNIK GMBH
Enge Gasse 1
D-8572 Auerbach/Opf. (DE)

(72) Erfinder : KLEIN, Franz
Wachberg 13, Willenberg
D-8570 Pegnitz (DE)
Erfinder : BAUMANN, Otto
Siechenstrasse 36
D-8572 Auerbach (DE)

(74) Vertreter : Hafner, Dieter Dr. Dipl.-Phys.
Essenweinstrasse 4-6
D-8500 Nürnberg70 (DE)

## Beschreibung

Die Erfindung betrifft eine Vorrichtung mit in einem Gehäuse angeordneter Lichtquelle, einem Ventilator zur Kühlung, Lufteintritts- und Luftaustrittsöffnungen, Lichtleitmittel, Filterelemente für das Licht und Mittel zum Polarisieren des Lichtes, insbesondere zur Stimulierung biologischer Prozesse, die mit der Zellaktivität zusammenhängen, insbesondere zur Unterstützung des Heilprozesses von Verletzungen der Körperoberfläche, wie Wunden, Ulcera und verschiedene epithele Schädigungen, aber auch für andere Anwendungen auf medizinischem, pharmazeutischen und biologischem Gebiet.

Es ist bereits ein Verfahren zur Stimulierung biologischer Prozesse bekannt, bei dem der zu stimulierende Bereich mit einem Lichtbündel vorbestimmter Intensität bestrahlt wird, und die Bestrahlung mit einem linear polarisierten nicht kohaerenten Licht erfolgt, das Wellenlängen-Komponenten oberhalb von 300 nm aufweist. Vorzugsweise hat das bestrahlende Licht eine kontinuierliche oder quasi kontinuierliche spektrale Verteilung wenigstens im Wellenlängenbereich von größer 300 nm. Bei einer Vorrichtung zur Erzeugung des benötigten Lichtes ist beispielsweise eine Lichtquelle vorgesehen, die inkohaerentes Licht mit Spektralkomponenten oberhalb von 300 nm abgibt, und ein Ablenksystem im Strahlengang des von der Lampe abgegebenen Lichtes um die Lichtstrahlen in eine gegebene Behandlungsrichtung zu bringen und im Strahlengang ein Polarisator um polarisierte Lichtstrahlen zu erzeugen.

Die Lichtquelle erzeugt, physikalisch bedingt, Wärme, die aus dem die Lichtquelle aufnehmenden Gehäuse abgeführt werden muß. Dazu ist ein Ventilator vorgesehen, der die Lichtquelle kühlt. Durch den Ventilator wird Luft in das Gehäuse eingesaugt bzw. aus dem Gehäuse ausgeblasen. Dadurch besteht die Gefahr einer Verschmutzung der optischen Einrichtung durch Staub oder einer Kontamination durch mit der Luft mitgerissene Bakterien, Viren oder dergl.

Der Erfindung liegt die Aufgabe zugrunde, eine Vorrichtung der eingangs erläuterten Art dahingehend auszubilden, daß Beeinträchtigungen und-/oder Störungen der optischen Einrichtungen und/oder Schädigungen des mit dem Licht zu behandelnden Individuums durch die vom Gerät erzeugte warme Luft bzw. die Kühlluft vermindert bzw. ausgeschlossen werden.

Diese Aufgabe wird nach der Erfindung bei der Vorrichtung der eingangs erläuterten Art im wesentlichen dadurch gelöst, daß an einem lufteintritts- und gleichzeitig lichtaustrittsseitigen Ende des Gehäuses ein auswechselbares, lichtdurchlässiges Luftfilterelement mit einer zentralen Lichtdurchtrittsöffnung angeordnet ist, die von einem ringförmig angeordnete Luftdurchtrittsöffnungen überdeckenden Luftfilter umgeben ist, daß die Haltemittel für die Filterelemente, Polarisationsmittel, Lichtquelle und Ventilator luftdurchlässig ausgebildet bzw. angeordnet sind und daß Führungsmittel für die durch die Lichtquelle erhitzte und vom Ventilator bewegte Luft und/oder die Kühlluft vorgesehen sind.

Es ist sichergestellt, daß das Filter weder den Lichtstrahl stört noch durch den Lichtstrahl beaufschlagt wird. Die vom Ventilator angesaugte Kühlluft streicht dann im wesentlichen an der Innenwand des Gehäuses entlang und verläßt das Gehäuses am rückwärtigen Ende.

In manchen therapeutischen Fällen oder auch anderen Anwendungen kann es vorteilhaft sein, die zu behandelnde Fläche nicht nur mit Licht zu bestrahlen, sondern auch zu erwärmen. Dazu kann in vorteilhafter Weise die von der lichtquelle erwärmte Luft verwendet werden, die dann vom Ventilator durch die vordere, luftaustrittsseitige, Öffnung des Gehäuses abgeblasen wird. In diesem Fall ist es vorteilhaft, wenn das Luftfilterelement bezüglich des Strahlenganges des Lichtes hinter der Lichtquelle angeordnet ist. In vorteilhafter Weise weist das Luftfilterelement ein sich über den Querschnitt des Gehäuses erstreckendes Filter auf.

Das jeweilige Luftfilter kann in verschiedener Weise realisiert sein. Bei einem ersten Ausführungsbeispiel besteht das Filter aus textilem Material. Bei einer Abwandlung der Erfindung besteht das Filter aus einem Drahtgewebe. In noch weiterer Abwandlung kann das Filter aus Kunststoff-Fasern oder dergl. bestehen.

Bei einem besonders vorteilhaften Ausführungsbeispiel ist das Luftfilterelement in einem auf das Gehäuse aufsetzbaren Ringhalter angeordnet.

Zur Vereinfachung der Verbindung zwischen Ringhalter und Gehäuse weist das Gehäuse eine Klemmnut für den Ringhalter auf.

Bei dem Ausführungsbeispiel, bei dem der Ventilator die von der Lichtquelle erwärmte Luft vorne, d. h. parallel mit dem Lichtstrahl ausbläst, ist das Luftfilterelement in einem innerhalb des Gehäuses vorgesehenen Ringhalter angeordnet.

Um den Ventilator in den Schutz gegen Verstaubung mit einzuschließen ist es vorteilhaft, wenn der Ringhalter zwischen Ventilator und lufteintrittsseitigem Gehäuseende angeordnet ist.

In zweckmäßiger Ausgestaltung der Vorrichtung besteht das Tragelement für die Lichtquelle aus einem Ringsockel mit Luftschlitzen. Dadurch wird erreicht, daß die Lichtquelle vollständig von der Kühlluft umströmt wird.

Gemäß einer besonderen konstruktiven Ausgestaltung der erfindungsgemäßen Vorrichtung ist das Tragelement für den Ventilator zugleich als Luftfilterelement ausgebildet.

In zweckmäßiger Weiterbildung sind die Halterungen für die optischen Elemente mit schlitzartigen Luftdurchtrittsöffnungen versehen.

Gemäß einem bevorzugten Ausführungsbeispiel der Erfindung bestehen die Haltemittel für die optischen Elemente und/oder die Tragelemente für die Lampe und/oder dem Ventilator aus in Abständen am Umfang des Gehäuseinneren angeordneten Haltenocken.

Insbesondere als Polarisationsmittel Spiegel oder dergl. verwendet werden, ist es vorteilhaft, wenn im Lichtweg hinter dem Polarisationselement ein Luftleitelement vorgesehen ist.

In zweckmäßiger Weiterbildung der Erfindung weist das Gehäuse eine zur Lichtaustrittsrichtung parallele, luftdicht verschlossene Durchblicköffnung auf.

Um sowohl eine Betriebsart durchführen zu können, bei der die Kühlluft am lichtaustrittsseitigem Ende des Gehäuses angesaugt wird als auch eine Betriebsart, bei der aus dem lichtaustrittsseitigem Ende Warmluft ausgeblasen wird, ist es vorteilhaft, wenn an beiden Endteilen des Gehäuses je ein Luftfilterelement angeordnet ist.

Für dieses Ausführungsbeispiel ist es ferner vorteilhaft, wenn der Ventilator umschaltbar als Lüfter und Gebläse ausgebildet ist.

Weitere Einzelheiten, Merkmale und Vorteile der Erfindung werden anhand der Zeichnung näher erläutert, die schematisch ein Ausführungsbeispiel darstellt.

Dabei zeigt :

Fig. 1 einen Querschnitt durch ein Ausführungsbeispiel einer erfindungsgemäßen Vorrichtung,

Fig. 2 eine Rückansicht in Richtung des Pfeiles A der Fig. 1,

Fig. 3 eine Ansicht in Richtung der Schnittlinie III-III der Figur 1,

Fig. 4 einen Teilausschnitt des Gehäuses mit aufgestecktem Luftfilterelement, und

Fig. 5 ein Ausführungsbeispiel eines Tragelementes.

In einem, beispielsweise zweiteilig ausgebildeten, Gehäuse mit einem vorderen Gehäuseteil 1 und einem rückwärtigem Gehäuseteil 2 ist eine Lichtquelle 3 angeordnet, die das für die beispielsweise Stimulierung biologischer Prozesse bestimmte Licht abgibt. Ferner sind Ilchtfilter, beispielsweise UV- und/oder IR-Filter 5, ein Polarisator, beispielsweise ein Brewster-Spiegel 6 sowie ein Ventilator 4 angeordnet. Ferner können Lichtleitmittel 11, Spiegel oder Linsen 32 vorgesehen sein. Im Gehäuse ist ferner eine elektrische Anschlußplatte 29 vorgesehen, die durch eine Kabelzuführungsöffnung 30 ein entsprechendes Kabel für die Netzversorgung angeschlossen ist.

Am rückseitigen Ende des Gehäuses 2 sind Kühlschlitze 28 angebracht. Die Gehäuseteile 1, 2 können vorzugsweise im Bereich der Lichtquelle 3 Kühlschlitze 27 aufweisen.

Das Gehäuse 2 bzw. Gehäuseteil 1 kann einen Handgriff 31 aufweisen bzw. mit einem Handgriff 31 einstückig ausgebildet sein. Anstelle des Handgriff 31 kann auch eine Haltevorrichtung für eine Stativbefestigung oder dergl. angebracht bzw. vorgesehen sein. Am lichtaustrittsseitigem Ende 7 des Gehäuses 1 ist eine Lichtleit bzw. Sammelvorrichtung, beispielsweise eine Linse 32 vorgesehen.

Zur Beobachtung des Behandlungsfeldes ist zweckmäßiger Weise am Gehäuseteil 1 eine Durchblicksöffnung 26 vorgesehen, deren Blickrichtung parallel zum Lichtstrahl verläuft. Die Durchblicksöffnung 26 ist luftdicht verschlossen.

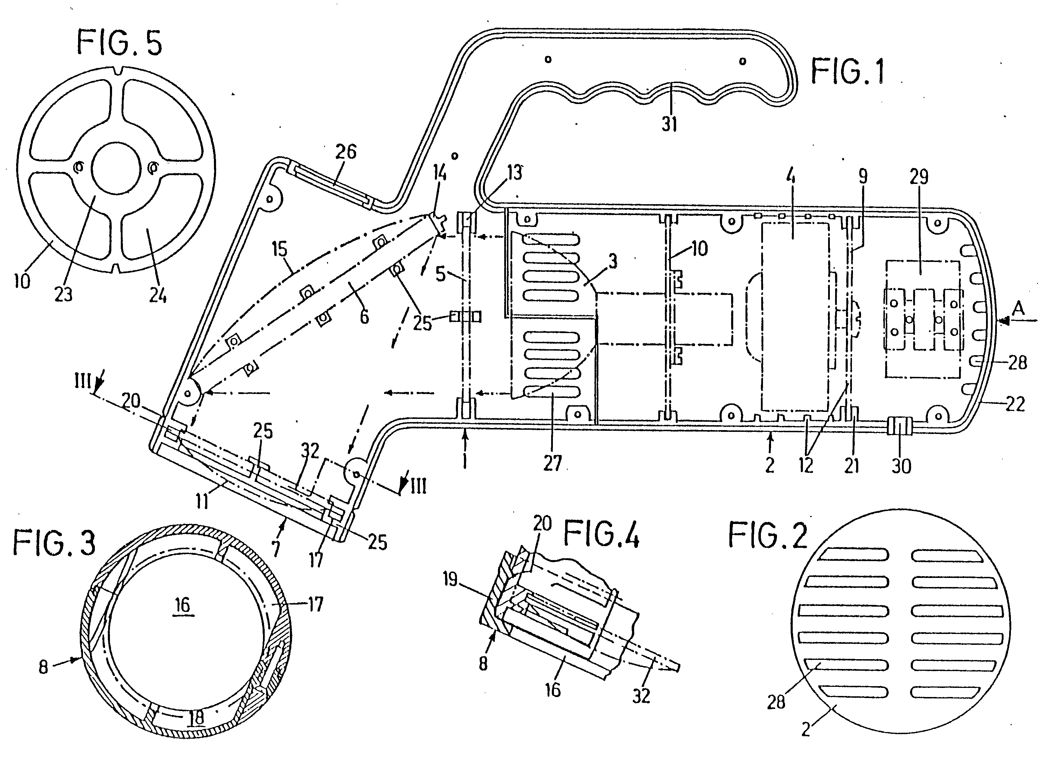

Um das Eindringen von Staub und/oder Bakterien oder dergl. zu verhindern, ist erfindungsgemäß am vorderen lichtaustrittsseitigem Ende 7 des Gehäuses 1 ein Luftfilterelement 8 angebracht. Dieses Luftfilterelement 8 besteht vorzugsweise, gemäß Fig. 3, aus einem Filter 18 der ringförmige Luftaustrittsöffnungen 17 überdeckt. Der ringförmige Teil 17 umgibt eine zentrale Lichtdurchtrittsöffnung 16. Die vom Ventilator 4 angesaugte Kühlluft strömt durch das, die ringförmige Luftdurchtrittsöffnung 17 abdeckende, Filter 18 und wird dabei entstaubt. Das Filter 18 kann aus textilem Material bestehen, beispielsweise einem feingewebtem Tuch, oder aus einem Metallgitter mit sehr feinen Gitteröffnungen, oder aus einem Kunststofffilter in Tuchform aus Fasern hergestellt oder aus feinem Granulat oder dergl. Bei der Verwendung von Textilgewebe besteht die Möglichkeit, dieses bei Gebrauch mit einer desinfizierenden Lösung zu beaufschlagen. Dabei wird sichergestellt, daß Viren und Bakterien oder dergl. nicht in das Gehäuse-Innere eindringen und diese kontaminieren können.

Durch die angesaugte Luft wird die Lichtquelle 3 gekühlt, wobei die erwärmte Luft durch die Kühlöffnungen 28 am hinteren Ende 22 des Gehäuses 2 ausgeblasen wird.

Um ein leichtes Auswechseln des Luftfilterelementes zu ermöglichen, ist es vorteilhaft, wenn das Element 8 in einem Ringhalter 19 angeordnet ist, der über das vordere Ende 7 des Gehäuses 1 geschoben werden kann. Zur sicheren Verbindung mit dem Gehäuse 1 ist es zweckmäßig, wenn das Gehäuse eine Klemmnut 20 aufweist (Fig. 4).

Ein Haltemittel 10 für die Lichtquelle 3 ist (Fig. 5) derart ausgebildet, daß ein Ringsockel 23 von Luftschlitzen 24 umgeben ist. Dadurch wird erreicht, daß die vom Ventilator 4 angesaugte Kühlluft bzw. die vom Ventilator 4 ausgeblasene Kühlluft die Lichtquelle 3 vollständig umfließen kann.

Die Halteelemente 13 für das Filterelement 5, 14 für den Polarisator 6 sind analog zum Luftfilterelement 8 mit einer zentralen Lichtdurchtrittsöffnung und ringförmigen Luftdurchtrittsöffnungen versehen.

Die Halteelemente können in ringförmigen Halterungen 21 befestigt sein, oder in einzelnen, über den Umfang des Gehäuses verteilten Haltenocken 25.

Die Haltemittel 12 für den Ventilator 4 können gleichzeitig, wie in Fig. 1 dargestellt, als luftfilterelement 9 ausgebildet sein.

Bei einer ersten Betriebsart der erfindungsgemäßen Vorrichtung wird vom Ventilator 4 am lichtaustrittsseitigem Ende 7 des Gehäuses 1 Luft durch ein vorderes Luftfilterelement 8 angesaugt und durch das Gehäuse, and der Lichtquelle 3 vorbei, durchgeleitet und am Gehäuseende durch die Kühlschlitze 28 abgeführt.

Bei einer zweiten Betriebsart saugt der Ventilator 4 Luft durch die Öffnungen 28 am rückwärtigen Teil an, bläst sie über die Lichtquelle 3 wobei die Luft erhitzt wird und drückt die Luft dann durch das Luftaustrittsseitige Ende 7, parallel zum Lichtstrahl, auf die zu behandelnde Fläche. Bei dieser zweiten Betriebsart wird der therapeutische Effekt der Lichtbestrahlung durch die lokale Erwärmung mittels der von der Lichtquelle 3 erhitzten Luft unterstützt.

Die Erfindung ist nicht auf das dargestellte und beschriebene Ausführungsbeispiel beschränkt. Bei Verwendung eines vom Licht geradlinig durchlaufenen Polarisator 6 kann das Gehäuse als geradliniges Rohr ausgebildet sein. Das Gehäuse kann einteilig sein. Die Kühlschlitze 27 können entfallen. Das Gehäuse kann kreisförmigen oder auch rechteckigen, quadratischen oder ovalen Querschnitt aufweisen. Die Luftführungsmittel 15 können, wenn sie aus durchsichtigem Material bestehen, auch vor dem Polarisator 6 angeordnet sein. Die Erfindung umfaßt somit alle fachmännischen Weiterbildungen und Abwandlungen sowie Teil- und/oder Unterkombinationen der beschriebenen und/oder dargestellten Merkmale und Maßnahmen.

**Patentansprüche**

1. Therapeutische Bestrahlungsvorrichtung mit in einem Gehäuse (1, 2) angeordneter Lichtquelle (3), einem Ventilator (4) zur Kühlung, Lufteintritts- und Luftaustrittsöffnungen, Lichtleitmitteln (11), Filterelementen (5) für das Licht und Mitteln (6) zum Polarisieren des Lichtes, zur Stimulierung biologischer Prozesse, die mit der Zellaktivität zusammenhängen, insbesondere zur Unterstützung des Heilprozesses von Verletzungen der Körperoberfläche, wie Wunden, Ulcera und verschiedene epithele Schädigungen, aber auch für andere Anwendungen auf medizinischem, pharmazeutischem und biologischem Gebiet, dadurch gekennzeichnet, daß an einem lufteintritts- und gleichzeitig lichtaustrittsseitigen Ende (7) des Gehäuses (1, 2) ein auswechselbares, lichtdurchlässiges Luftfilterelement (8) mit einer zentralen Lichtdurchtrittsöffnung (16) angeordnet ist, die von einem ringförmig angeordnete Luftdurchtrittsöffnungen (17) überdeckenden Luftfilter (18) umgeben ist, daß luftdurchlässige Haltemittel (10, 12, 13 und 14) für die Lichtquelle (3), den Ventilator (4), die Lichtfilterelemente (5) und die Polarisationsmittel (6) vorgesehen sind und daß im Gehäuse (1) Luftführungsmittel (15) für die durch die Lichtquelle (3) erhitzte und vom Ventilator (4) bewegte Luft und/oder die Kühlluft im Bereich zwischen Lichtquelle (3) und Gehäuseende (7) angeordnet sind.

2. Vorrichtung nach Anspruch 1, dadurch gekennzeichnet, daß das Luftfilterelement (8) in einem auf das Gehäuse (1) aufsetzbaren Ringhalter (19) angeordnet ist.

3. Vorrichtung nach Anspruch 2, dadurch gekennzeichnet, daß das Gehäuse (1) eine Klemmnut (20) für den Ringhalter (19) aufweist.

4. Vorrichtung nach einem der Ansprüche 1-3, dadurch gekennzeichnet, daß das Luftfilter (18) aus textilem Material besteht.

5. Vorrichtung nach einem der Ansprüche 1-3, dadurch gekennzeichnet, daß das Luftfilter (18) aus einem Drahtgewebe besteht.

6. Vorrichtung nach einem der Ansprüche 1-3, dadurch gekennzeichnet, daß das Luftfilter (18) aus Kunststoff-Fasern besteht.

7. Vorrichtung nach Anspruch 1 oder folgenden, dadurch gekennzeichnet, daß das Haltemittel (10) für die Lichtquelle (3) aus einem Ringsockel (23) mit Luftschlitzen (24) besteht.

8. Vorrichtung nach Anspruch 1 oder folgenden, dadurch gekennzeichnet, daß das Haltemittel (12) für den Ventilator (4) als Luftfilterelement ausgebildet ist.

9. Vorrichtung nach Anspruch 1 oder folgenden, dadurch gekennzeichnet, daß die Haltemittel (13, 14) für die optischen Elemente (5, 6) mit schlitzartigen Luftdurchtrittsöffnungen versehen sind.

10. Vorrichtung nach einem der Ansprüche 1-8, dadurch gekennzeichnet, daß die Haltemittel (13, 14) für die optischen Elemente (5, 6) und/oder die Haltemittel (10, 12) für die Lichtquelle (3) und/oder für den Ventilator (4) aus in Abständen am Umfang des Gehäuseinneren angeordneten Haltenocken (25) bestehen.

11. Vorrichtung nach einem der Ansprüche 1-10, dadurch gekennzeichnet, daß im Lichtweg hinter dem Polarisator (6) die Luftführungsmittel (15) angeordnet sind.

12. Vorrichtung nach einem der Ansprüche 1-11, dadurch gekennzeichnet, daß das Gehäuse (1) eine zur Lichtaustrittsrichtung parallele, luftdicht verschlossene Durchblicköffnung (26) aufweist.

13. Vorrichtung nach einem der vorhergehenden Ansprüche, dadurch gekennzeichnet, daß im Gehäuse (2) ein Luftfilterelement (9) vorgesehen ist, das ein sich über den Querschnitt des Gehäuses (2) erstreckendes Filter aufweist.

14. Vorrichtung nach Anspruch 13, dadurch gekennzeichnet, daß das Luftfilterelement (9) in einem innerhalb des Gehäuses (2) vorgesehenen Ringhalter (21) angeordnet ist.

15. Vorrichtung nach Anspruch 14, dadurch gekennzeichnet, daß der Ringhalter (21) zwischen Ventilator (4) und einem lufteintrittsseitigen Gehäuseende (22) angeordnet ist.

16. Vorrichtung nach einem der vorhergehenden Ansprüche, dadurch gekennzeichnet, daß an beiden Enden (7 bzw. 22) des Gehäuses (1, 2) je ein Luftfilterelement (8 bzw. 9) angeordnet ist.

17. Vorrichtung nach Anspruch 16, dadurch gekennzeichnet, daß der Ventilator (4) umschaltbar als Lüfter und Gebläse ausgebildet ist.

18. Vorrichtung nach einem der vorhergehenden Ansprüche, dadurch gekennzeichnet, daß die Polarisationsmittel (6) durch einen Brewster-Spiegel gebildet sind.

**Claims**

1. Therapeutic radiation device with a source of light (3) disposed in a housing (1, 2), a fan (4) for cooling, air inlet and outlet openings, light conducting means (11), filter elements (5) for the light and means (6) for the polarization of the light, for stimulating biological processes connected with cell activity, in particular for supporting the healing process of wounds on the surface of the body, such as injuries, ulcers and epithelic lesions, but also for other applications in the medical, pharmaceutical and biological fields, characterised in that at one air inlet and at the same time air outlet end of the housing (1, 2) a removable, light-permeable air filter element (8), having a central passage opening (16) for light, is disposed surrounded by an air filter (18) covering annularly disposed air passage openings (17), in that air-permeable holding means (10, 12, 13 and 14) are provided for the source (3) of light, the fan (4), the light filter elements (5) and the polarization means (6), and in that in the housing (1) air guiding means (15) are provided for the air heated by the light source (3) and moved by the fan (4) and/or for the cooling air in the region between the spurce (3) of light and the end (7) of the housing.

2. Device according to Claim 1, characterised in that the air filter element (3) is disposed in a ring holder (19) which can be placed onto the housing (1).

3. Device according to Claim 2, characterised in that the housing (1) has a clamping groove (20) for the ring holder (19).

4. Device according to one of Claims 1 to 3, characterised in that the air filter (18) is of textile material.

5. Device according to one of Claims 1 to 3, characterised in that the air filter (18) is in a wire fabric.

6. Device according to one of Claims 1 to 3, characterised in that the air filter (18) is made of plastic fibres.

7. Device according to Claim 1 or the following, characterised in that the holding means (10) for the source (3) of light consists in a ring base (23) with air slits (24).

8. Device according to Claim 1 or the following, characterised in that the holding means (12) for the fan (4) is constructed as an air filter element.

9. Device according to Claim 1 or the following, characterised in that the holding means (13, 14) for the optical elements (5, 6) are provided with slit-like air passage openings.

10. Device according to one of Claims 1 to 8, characterised in that the holding means (13, 14) for the optical elements (5, 6) and/or the holding means (10, 12) for the source (3) of light, and/or for the fan (4) consist of holding cams (25) disposed at intervals on the perimeter of the inside of the housing.

11. Device according to one of Claims 1 to 10, characterised in that the air ducting means (15) are disposed along the path of light behind the polarizer (6).

12. Device according to one of Claims 1 to 11, characterised in that the housing (1) has an airtightly closed inspection port (26) parallel with the direction of light outflow.

13. Device according to one of the foregoing claims characterised in that in the housing (2) an air filter element (9) is provided which has a filter extending over the cross-section of the housing (2).

14. Device according to Claim 13, characterised in that the air filter element (9) is disposed in a ring holder (21) provided inside the housing. (2).

15. Device according to Claim 14, characterised in that the ring holder (21) is disposed between the fan (4) and a housing and (22) on the air inlet side.

16. Device according to one of the foregoing claims, characterised in that an air filter element (8, 9) is disposed at each end (7, 22) of the housing (1, 2).

17. Device according to Claim 16, characterised in that the fan (4) is constructed reversibly as extractor and blower.

18. Device according to one of the foregoing claims, characterised in that the polarization means (6) are constituted by a Brewster mirror.

**Revendications**

1. Dispositif d'irradiation thérapeutique comportant une source de lumière (3) disposée dans un boîtier (1, 2), un ventilateur (4) de refroidissement, des ouvertures d'entrée d'air et de sortie d'air, des moyens de guidage (11) de la lumière, des organes de filtration (5) de la lumière et des moyens (6) pour polariser la lumière, ce dispositif étant destiné à stimuler des processus biologiques qui sont liés à l'activité cellulaire, en particulier à favoriser le processus de guérison de blessures superficielles, telles que plaies, ulcères et diverses lésions de l'épithélium, mais aussi à d'autres applications dans les domaines médical, pharmaceutique et biologique, caractérisé en ce qu'un organe interchangeable de filtration (8) de l'air, perméable à la lumière et comportant en son centre une ouverture de passage (16) de la lumière, est disposé à une extrémité (7) du boîtier (1, 2) correspondant à l'entrée de l'air ainsi qu'à la sortie de la lumière, laquelle ouverture est entourée d'un filtre à air (18) recouvrant des ouvertures de passage (17) de l'air disposées en cercle, en ce que des supports (10, 12, 13 et 14) perméables à l'air sont prévus pour la source de lumière (3), le ventilateur (4), les organes de filtration (5) de la lumière et les moyens de polarisation (6) et en ce que des moyens de canalisation (15) de l'air, destinés à l'air de refroidissement et/ou à l'air chauffé par la source de lumière (3) et mis en mouvement par le ventilateur (4), sont disposés dans le boîtier (1), dans l'espace entre la source de lumière (3) et l'extrémité (7) du boîtier.

2. Dispositif selon la revendication 1, caractérisé en ce que l'organe de filtration (8) de l'air est placé dans un support annulaire (19) qui est agencé de façon à pouvoir être adapté sur le boîtier (1).

3. Dispositif selon la revendication 2, caractérisé en ce que le boîtier (1) comprend une gorge d'encastrement (20) destinée au support annulaire (19).

4. Dispositif selon l'une des revendications 1 à 3, caractérisé en ce que le filtre à air (18) est composé d'une matière textile.

5. Dispositif selon l'une des revendications 1 à 3, caractérisé en ce que le filtre à air (18) est constitué d'une toile métallique.

6. Dispositif selon l'une des revendications 1 à 3, caractérisé en ce que le filtre à air (18) est constitué de fibres de matière plastique.

7. Dispositif selon la revendication 1 ou l'une des suivantes, caractérisé en ce que le support (10) de la source de lumière (3) est constitué d'une base annulaire (23) comportant des fentes (24) pour l'air.

8. Dispositif selon la revendication 1 ou l'une des suivantes, caractérisé en ce que le support (12) du ventilateur (4) est agencé en organe de filtration de l'air.

9. Dispositif selon la revendication 1 ou l'une des suivantes, caractérisé en ce que les supports (13, 14) des éléments optiques (5, 6) comportent des ouvertures en forme de fente pour le passage de l'air.

10. Dispositif selon l'une des revendications 1 à 8, caractérisé en ce que les supports (13, 14) des éléments optiques (5, 6) et/ou les supports (10, 12) de la source de lumière (3) et/ou du ventilateur (4) sont constitués de saillies de maintien (25) disposées à distance les unes des autres sur le pourtour intérieur du boîtier.

11. Dispositif selon l'une des revendications 1 à 10, caractérisé en ce que les moyens de canalisation (15) de l'air sont placés sur le trajet optique, derrière le polariseur (6).

12. Dispositif selon l'une des revendications 1 à 11, caractérisé en ce que le boîtier (1) comprend une fenêtre de visée (26) parallèle à la direction de sortie de la lumière et étanche à l'air.

13. Dispositif selon l'une des revendications précédentes, caractérisé en ce qu'il est prévu, dans le boîtier (2), un organe de filtration (9) de l'air qui comprend un filtre couvrant la section du boîtier (2).

14. Dispositif selon la revendication 13, caractérisé en ce que l'organe de filtration (9) de l'air est disposé dans un support annulaire (21) prévu à l'intérieur du boîtier (2).

15. Dispositif selon la revendication 14, caractérisé en ce que le support annulaire (21) est disposé entre le ventilateur (4) et une extrémité (22) du boîtier située du côté de l'entrée de l'air.

16. Dispositif selon l'une des revendications précédentes, caractérisé en ce qu'un organe de filtration (8 ou 9) de l'air est disposé à chacune des deux extrémités (7 ou 22) du boîtier (1, 2).

17. Dispositif selon la revendication 16, caractérisé en ce que le ventilateur (4) est agencé de manière à pouvoir être actionné en ventilateur aspirant et en ventilateur soufflant.

18. Dispositif selon l'une des revendications précédentes, caractérisé en ce que les moyens de polarisation (6) sont formés par un miroir de Brewster.

FIG.5

FIG.1

31

26

14

13

4

9

29

15

5

3

10

A

6

25

28

III

22

20

III

25

32

27

2

12

21

30

11

7

17

25

III

20

FIG.4

FIG.2

19

8

16

32

28

FIG.3

16

17

8

18

10

23

24

0 137 005